Europäisches Patentamt

European Patent Office

Office européen des brevets

Publication number: **0 367 166**
A1

# EUROPEAN PATENT APPLICATION

Application number: 89120061.0

Date of filing: 28.10.89

Int. Cl.5: **A61K 37/02 , A61K 47/48 , A61K 39/395** .

The applicant has filed a statement in accordance with Rule 28 (4) EPC (issue of a sample only to an expert). Accession number(s) of the deposit(s): FERM BP-852.

Claims for the following Contracting States: ES + GR.

Priority: 31.10.88 JP 276917/88

Date of publication of application: 09.05.90 Bulletin 90/19

Designated Contracting States:
AT BE CH DE ES FR GB GR IT LI LU NL SE

Applicant: TAKEDA CHEMICAL INDUSTRIES, LTD.
3-6, Doshomachi 2-chome Chuo-ku
Osaka 541(JP)

Inventor: Igari, Yasutaka
3-D, 9-29, Uozakinakamachi 4-chome
Higashinada-ku
Kobe Hyogo 658(JP)
Inventor: Doken, Kazuhiro
11-10, Abikohigashi 3-chome Sumiyoshi-ku
Osaka 558(JP)
Inventor: Sato, Jun
19-301, 5 Aoki-cho Nishinomiya
Hyogo 662(JP)

Representative: von Kreisler, Alek et al
Patentanwälte Von
Kreisler-Selting-Werner-Schönwald-Fues-Da-
llmeyer Deichmannhaus
D-5000 Köln 1(DE)

## Modified interleukin-2 and production thereof.

A modified interleukin-2 (IL-2), in which IL-2 and immunoglobulin or its fragment having an antibody activity are chemically bonded with each other, exhibits its IL-2 activity for a long time when administered to a living body, and the modified IL-2 can be advantageously used as a low-clearance drug for injection.

EP 0 367 166 A1

## MODIFIED INTERLEUKIN-2 AND PRODUCTION THEREOF

The present invention relates to a modified interleukin-2 which is very stable in a living body.

Interleukin-2 (hereinafter also abbreviated IL-2), a soluble protein produced by T cells activated with lectin or antigen, potentiates both humoral and cell-mediated immunity, thus serving well for the purpose of normalizing the state of depressed immunity. This immune activity of IL-2 is useful for the treatment of cancer, bacterial or viral infection, autoimmune diseases, immunodeficiency etc.

However, IL-2 disappears rapidly in a living body, as reported that its half life in blood in administration to humans is 6.9 minutes [M. Taotze et al., Journal of Immunology, *135*, 2865-2875 (1985)]; therefore, IL-2 is thought to exhibit no satisfactory action, or requires high doses, in clinical treatment situations.

In view of this fact, a method of increasing the stability of IL-2 in blood was proposed in which IL-2 is bound with polyethylene glycol (Nishimura et al., Japanese Patent unexamined Publication No. 226821/1985 or N. Katre et al., Japanese Patent unexamined PublicationNo. 503171 1987).

Meantime, Kawase et al. have recently reported that the antitumor effect of IL-2 is enhanced by using IL-2 in combination with anticancer monoclonal antibody (Kawase et al., Cancer Research, *48*, 1173-1179(1988)) ("monoclonal antibody" is hereinafter also abbreviated Mab).

If the stability of IL-2 in blood can be increased without spoiling its bioactivities, it will be possible to enhance its desired effect and mitigate its adverse action due to mass administration.

The present inventors made investigations of modified IL-2 that maintains the bioactivities of IL-2 and that possesses increased IL-2 stability in a living body, and found that in IL-2 administration to a living bodys, the stability and retention time of IL-2 in a living body can be increased by using it in a conjugate with immunoglobulin (hereinafter also abbreviated Ig) or its fragment. The present inventors made further investigations based on this finding, and have developed the present invention.

Accordingly, the present invention provides (1) a modified interleukin-2 in which interleukin-2 and immunoglobulin or its fragment having an antibody activity are chemically bound with each other, (2) a method for agent an amino group of interleukin-2 which comprises binding via a crosslinking agent an amino group of interleukin-2 and a mercapto group resulting from reduction of immunoglobulin or its fragment having an antibody activity, (3) a method for producing modified interleukin-2 which comprises binding via a crosslinking agent an amino group of interleukin-2 and a thiol group which has been introduced to the amino group of immunoglobulin or its fragment having an antibody activity, (4) a modified interleukin-2 obtained by binding via a crosslinking agent an amino group of interleukin-2 and a mercapto group resulting from reduction of immunoglobulin or its fragment having an antibody activity, (5) a modified interleukin-2 obtained by binding via a crosslinking agent an amino group of interleukin-2 and thiol group which has been introduced to the amino group of immunoglobulin or its fragment having an antibody activity.

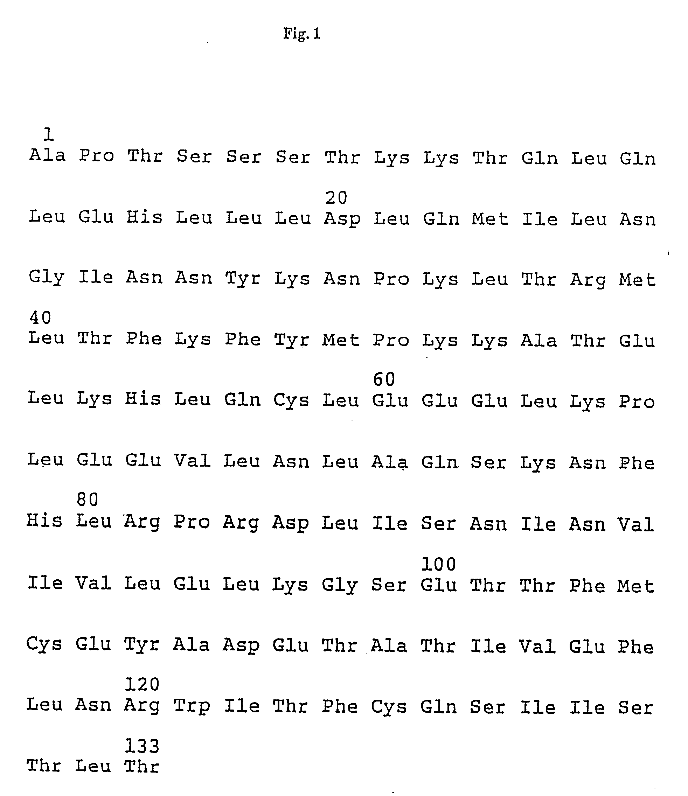

Examples of the IL-2 for the present invention includes substances possessing activity similar to that of IL-2, i.e., substances possessing action that permits subculture of T cells while maintaining their function. Specifically, mention may be made of polypeptide (I) (human IL-2), having the amino acid sequence shown in Fig. 1, and polypeptides comprising its partial amino acid sequence essential for its biological or immunological activities. Examples of such polypeptides include polypeptides lacking one amino acid residue from the amino terminal of polypeptide (I) (EPC Patent Publication No. 91539), polypeptides lacking four amino acid residues from the amino terminal of polypeptide (I) (Japanese Patent unexamined Publication No. 126088/1985) and polypeptides lacking several amino acid residues from the carboxyl terminal of polypeptide (I). Also included are polypeptides resulting from the partial elimination or substitution by other amino acid residue of the constituent amino acids of polypeptide (I), that has the amino acid sequence shown in Fig. 1, such as the polypeptide resulting from substitution of the 125-position cysteine residue by a serine residue (Japanese Patent unexamined Publication No. 93093/1984 which corresponds to U.S. Patent No. 4,518,584).

It is especially preferable to use human IL-2 having the amino acid sequence shown in Fig. 1 for the present invention. In this case the human IL-2 may be a mixture of human IL-2 having an additional methionine residue (Met) in the amino terminal and that having no additional Met (Japanese Patent unexamined Publication Nos. 115528/1985 and 78799/1986) or may have an alanine residue (Ala) in place of Met in the amino acid terminal (Japanese Patent unexamined publication No. 78799/1986).

The human IL-2 may also have a sugar chain.

Said Japanese Patent unexamined Publication No. 115528/1985 corresponds to European Patent Publication No. 145,390, and said Japanese Patent unexamined Publication No. 78799/1986 corresponds to European Patent Publication No.

176,299.

The Ig for the present invention may be of human derivation or animal derivation (e.g. mouse antitumor antibody obtained by immunizing a mouse with human tumor). The Ig may be polyclonal or monoclonal antibody. Examples of the Ig include the four subclasses of human blood component IgG, i.e., human IgG1 through IgG4, as well as IgA, IgE and IgD. IgG is especially preferable. In addition, the Ig may be intact, or may be a fragment having an antibody activity such as Fab, Fab' or F(ab')z. As the Ig or its fragment, it is preferable that Ig or its fragment has a group which changes to SH group by reduction. The SH group to be bound with a crosslinking agent may be one which is introduced by a thiol group-introducing agent.

Particularly, fragments of Ig having an antibody activity such as Fab, Fab' or F(ab')2 is preferable, and Fab' or F(ab')2 is more preferable, because when the fragment is employed the generating of an antibody against the present modified IL-2 is decreased, and therefore the IL-2 activity becomes sustained.

In binding the IL-2 with IgG or its fragment having an antibody activity (hereinafter also briefly referred to as fragment), a crosslinking agent maybe used.

Examples of the crosslinking agent include for example EMCS type such as N-(ε-maleimidocaproyloxy) succinimide (EMCS), 3-maleimidobenzoic acid N-hydroxysuccinimide (MBS), m-maleimidobenzoylsulfosuccinimide ester (sulfo-MBS), N-(3-maleimidopropionyloxy)succinimide, N-(γ-maleimidobutyryloxy)succinimide (GMBS), N-(β-maleimidopropyonyloxy) succinimide (BMPS), succinimidyl 4-(N-maleimidomethyl)cyclohexane-1-carboxylate (SMCC), sulfosuccinimidyl 4-(N-maleimidomethyl) cyclohexane1-carboxylate (sulfo-SMCC), succinimidyl 4-(p-maleimidophenyl)-butyrate (SMPB), sulfosuccinimidyl 4-(p-maleimidophenyl)butyrate (sulfo-SMPB), SPDP type such as N-succinimidyl 3-(2-pyridyldithio)-propionate (SPDP), SIAB type such as N-succinimidyl (4-iodoacetyl) aminobenzoate (SIAB), sulfosuccinimidyl (4-iodoacetyl) aminobenzoate (sulfo-SIAB). In the crosslinking agent, EMCS is more preferable.

Examples of the thiol group-introducing agent which introduces a thiol group to an amino group of immunoglobulin or its fragment include N-succinimidyl-s-acetylthioacetate (SATA), succinimidyl-3-(2-pyridylthio) propionate (SPDP), s-acetylmercaptosuccinic anhydride (SAMSA). In the thiol group-introducing agent, SATA is more preferable.

To emerge the mercapto group by reducing the immunoglobulin or its fragment having an antibody activity, the reaction is carried out by using a reducing agent such as dithiothreitol (DTT), 2-mercaptoethanol, ethanolamine at a temperature of between about 0 to 50°C. The solvents used in the reaction are for example generally an aqueous buffer solution of pH not less than 7.0. The concentration of the reducing agent in the solvent is for example about 0.5 mM to 50 mM.

The introduction of the thiol group to the amino group of IgG or its fragment is carried out by reacting IgG or its fragment with a thiol group-introducing agent. It is preferable that the ratio of the thiol group-introducing agent to IgG or its fragment is about 1:1 to about 100:1. The concentration of the solution of the thiol group-introducing agent is preferably a higher one, and more preferably about 0.01 to 100 μmol/mℓ. The reaction temperature is preferably at about 0 to 50°C, more preferably about 10 to 30°C.

For bonding via a crosslinking agent an amino group of IL-2 and mercapto group resulting from reducing immunoglobulin or its fragment, when EMCS type crosslinking agent is used, the amino group of IL-2 is maleimidated. The maleimidation is carried out by reacting IL-2 and the crosslinking agent under gentle stirring. It is preferable that the ratio of the crosslinking agent to IL-2 is about 1:1 to 100:1. The concentration of EMCS in the solution of the reaction system is preferably about 0.5 to 20 μmol/mℓ. The reaction temperature is preferably at about 0 to 50°C, more preferably about 10 to 30°C. The reaction time is preferably about 20 to 60 minutes.

For reacting thus obtained compound with the mercapto group, it is preferable that the ratio of the maleimidated compound to immunoglobulin or its fragment from which mercapto group is resulted is preferably about 1 : 1 to 5 : 1. The reaction temperature is preferably about 0 to 50°C. The reaction time is preferably about 20 to 60 minutes.

When SPDP type corsslinking agent is employed, pyridinedisulfide group is introduced to the amino group of IL-2, and then mercapto group is reacted with the introduced pyridinedisulfide group. The reaction of introducing pyridinedisulfide group the the amino group of IL-2 is carried out by reacting IL-2 and the crosslinking agent. The ratio of the crosslinking agent to IL-2 is preferably about 1:1 to 100:1. The concentration of SPDP type crosslinking agent in the solution of the reaction system is preferably about 0.5 to 20 μmol/mℓ. The reaction temperature is preferably about 0 to 50°C, more preferably about 10 to 30°C. The reaction time is preferably about 20 to 60 minutes.

In the reaction of thus produced compound with a mercapto group, the produced compound to which pyridinedisulfide group is introduced is reacted under gentle stirring with immunoglobulin or its fragment. The ratio of the produced compound to the immunoglobulin or its fragment is preferably about 1:1 to 5:1. The reaction temperature is normally about 0°C to 50°C, and the reaction time is preferably about 20 to 60 minutes.

When SIAB type crosslinking agent is employed, an active halogen group is introduced to the amino group of IL-2 by reacting IL-2 with the crosslinking agent, and the active halogen group is reacted with mercapto group. In the reaction of IL-2 with the crosslinking agent, the ratio of the crosslinking agent to IL-2 is preferably about 1:1 to 100:1. The reaction temperature is normally about 0° to 50°C, preferably about 10° to 30°C, and the reaction temperature is preferably about 20 to 60 minutes.

Thus obtained compound is reacted with the mercapto group under gentle stirring. The ratio of thus obtained compound to immunoglobulin or its fragment in which a mercapto group is resulted is preferably about 1:1 to 5:1. The reaction temperature is normally about 0° to 50°C, and the reaction time is preferably about 20 to 60 minutes.

The reaction of the amino group of IL-2 via a crosslinking agent with the thiol group introduced to the amino group of immunoglobulin or its fragment having an antibody activity is carried out by the same conditions of the reaction of the amino group of IL-2 via a crosslinking agent with the mercapto group resulting from a reduction mentioned above.

A modified IL-2 comprising IL-2 which is chemically bound with immunoglobulin or its fragment having antibody activity is thus obtained.

The modified IL-2 thus obtained can be used in a sustained-release injection. It is also possible to use the modified IL-2 in a composition for sustained-release injection by preparing it in combination with various carriers.

Since its toxicity is low, the modified IL-2 of the present invention can be used for the same purposes, in the same subjects, in the same manner, and at the same doses, as those for known IL-2. When sustained release is desired, an effect may be obtained with a dose of about 1/1 to 1/100 of the total dosage amount corresponding to the sustained-release period.

The IL-2 used for the present invention are low in toxicity. For example, the IL-2 which is produced by gene engineering techniques, which is purified by the method described in Japanese Patent unexamined Publication No. 115528/1985, which is then separated by the method described in Japanese Patent unexamined Publication No. 78799/1986, whose amino acid sequence is shown in Fig. 1, and whose specific activity is about $3.5 \times 10^4$ units/mg, when intravenously administered to cynomolgus monkeys, causes no death by 6 mg/kg single administration. As stated above, the IL-2 are low in toxicity, and thus can be safely administered.

Ig and its fragments are low in toxicity; modified IL-2, a conjugate of Ig or its fragment, is also low in toxicity.

When administered to living bodies, the modified IL-2 of the present invention is expected to exhibit a sustained-release effect of IL-2 because its metabolism and excretion are slow. It is therefore acceptable to administer the modified IL-2 in amounts exceeding ordinary dose levels of about 2 to 100 U·Kg as IL-2 in parenteral administration; but it is enough to employ the lower amount than the ordinary dose levels.

Note that 1 U (unit) represents the arbitrarily determined IL-2 activity per mℓ standard preparation, equaling about 28.6 ng of pure recombinant IL-2. Determination of IL-2 activity and definition of 1 unit are based on the method described in Japanese Patent unexamined Publication No. 115528/1985.

The modified IL-2 preparation of the present invention can be used to prevent and treat immune diseases and tumors in mammals (e.g. mice, cats, dogs, bovines, horses, sheep, goats, rabbits, humans).

Having increased stability, the modified IL-2 of the present invention can be administered in the form of an injection composition.

The injection composition may be formulated as appropriate with an IL-2 activity substance stabilizer [HSA (human serum albumin)], isotonizer (e.g. sodium chloride, glucose, mannitol, sorbitol), pH adjuster (e.g. hydrochloric acid, sodium hydroxide), stabilizer (e.g. sodium pyrosulfite, Rongalit, sodium hydrogen methasulfite), soothing agent (e.g. xylocaine hydrochloride, chlorobutanol, procaine hydrochloride), preservative (e.g. benzyl alcohol, phenol, methyl paraoxybenzoate, propyl paraoxybenzoate) and other additives to improve injection properties.

When a composition containing the modified IL-2 of the present invention is injected, the modified IL-2 is retained in blood for a long period, so that bioactivity of IL-2 is maintained for a long period. Therefore, the modified IL-2 of the present invention can be advantageously used in pharmaceutical preparations, particularly low-clearance protein. An injection preparation thus obtained can, for example, be administered intravenously, subcutaneously, intramuscularly, intratumorally or otherwise locally administered.

Brief Explanation of the Drawings

Fig. 1 shows the amino acid sequence of human IL-2 whose amino terminal is H-Ala-.

Fig. 2 shows the gel filtration pattern of the conjugate obtained in Example 1(1).

Fig. 3 shows the results of EIA determination of blood concentration of the conjugate obtained in Example 1 (1), obtained in Example 1 (2).

The IL-2 used in Examples below is a mixture of an IL-2 with methionine at its N-terminal and IL-2 having no N-terminal methionine, produced in the manner described in Japanese Patent unexamined Publication No. 115528/1985 or 78799/1986 using transformant Escherichia coli N4830/pTB285 (IFO 14437, FERM BP-852).

The above-mentioned transformant Escherichia coli N4830 pTB285 has been deposited under an accession number of IFO 14437 at the Institute for Fermentation, Osaka (IFO) since April 25, 1985. This microorganism has also been deposited under an accession number of FERM P-8199 at the Fermentation Research Institute, Agency of Industrial Science and Technology, Ministry of International Trade and Industry (FRI), Japan since April 30, 1985, and the deposit has been changed to the deposit under the Budapest Treaty and stored at the institute (FRI) under an accession number of FERM BP-852.

The abbreviations for amino acids used in the present specification and drawings are based on abbreviations specified by the IUPAC-IUB Commission on Biochemical Nomenclature or those commonly used in relevant fields. Note that when there is a possibility of the existence of an optical isomer in a particular amino acid, it is an L-form unless otherwise stated.

The present invention is hereinafter described in more detail by means of the following examples, but these examples are not to be construed as limitations on the invention.

Example 1

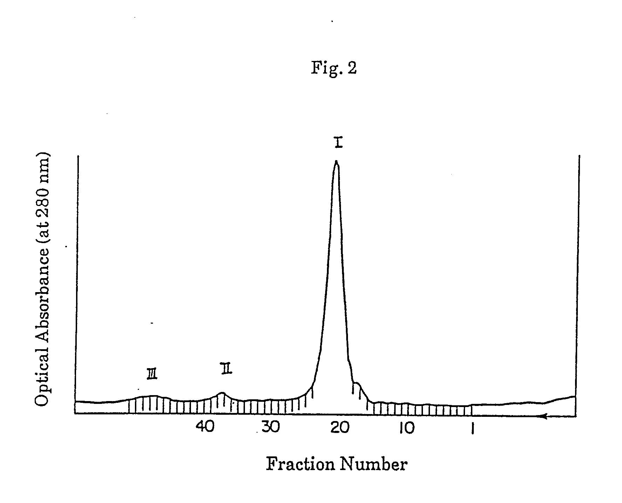

(1) To 0.5 mℓ of a phosphate-buffered physiological saline solution containing 2.0 mg/mℓ IL-2 as obtained by gene engineering techniques, 5 μℓ of a 40 mg/mℓ EMCS solution in tetrahydrofuran (THF) was added drop-by-drop while stirring, to maleimidate the IL-2. The excess portion of EMCS was removed using Sephadex G-25. Separately, to 1 mℓ of an aqueous solution of 0.05 mol potassium dihydrogen phosphate (pH 8.0) containing 3 mmol dithiothreitol (DTT) and 1.5 mmol EDTA, 0.5 mℓ of an aqueous solution of 18 mg/mℓ human IgG was added while stirring, to reduce the human IgG. The excess portion of low molecular compounds was removed using Sephadex G-25. To the solution of reduced IgG, the solution of maleimidated IL-2 was added, followed by stirring at room temperature for 2 hours. The mixed solution was then left at 4°C overnight and subjected to gel filtration through sephacryl S-200 to fractionate the conjugate (Fig. 2). In Fig. 2, I, II and III respectively show the peak of IL-2-IgG conjugate, peak of the product of decomposition and peak of IL-2. The molecular weight of the conjugate was estimated by SDS PAGE; a band appeared at a position corresponding to a molecular weight of about 150,000. This conjugate was found to retain the EIA activity of IL-2. Also, bioactivity determination revealed that the conjugate possesses bioactivities, as assessed on the basis of incorporation of 3-(4.5-dimethylthiazol-2-yl)-2.5-diphenyltetrazolium bromide (MTT), a pigment, due to NKC3 cell proliferation.

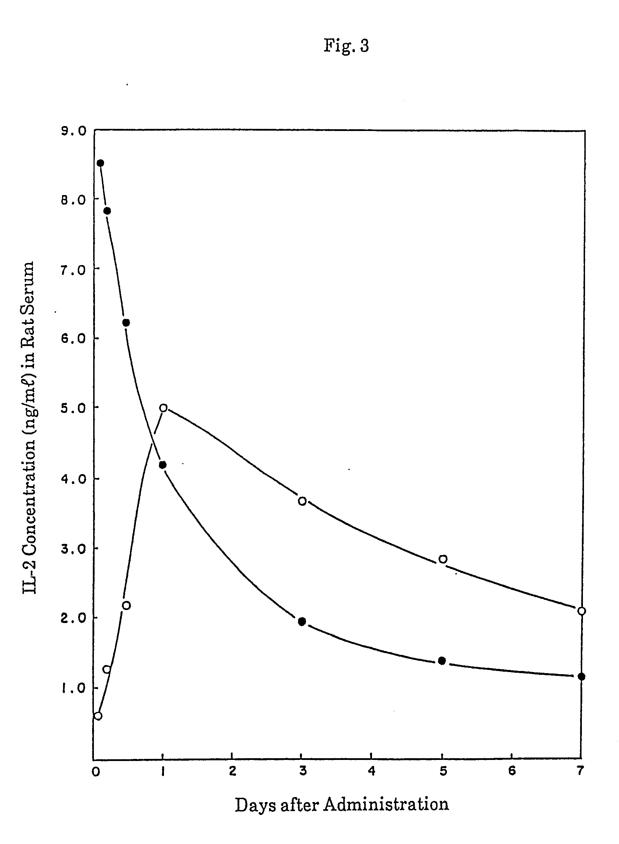

(2) The conjugate obtained in (1) above was administered subcutaneously or intravenously to rats in an amount of 75 ng, as calculated as IL-2, followed by daily blood sampling. The blood IL-2 concentration in the blood samples was determined by EIA; IL-2 was detected over a period of 7 days (Fig. 3). In Fig. 3, —●— and —○— respectively indicate the results of intravenous administration and those of subcutaneous administration. When the aqueous solution of IL-2 was intravenously administered to rats, IL-2 became undetectable in blood 6 hours later. Meantime, the IgG-IL-2 conjugate showed a good sustained-release effect over a long period.

Example 2

A conjugate was synthesized in the manner described in Example 1 but IgG monoclonal antibody against mouse B-16 melanoma (hereinafter also simply referred to as "anti-B16 melanoma antibody") was used in place of human IgG. This conjugate exhibited the EIA activity of IL-2.

Note that the IgG monoclonal antibody against mouse B-16 melanoma was produced as follows:

An anti-B16-melanoma-antibody-producing hybridoma (MM2-9B6) [this hybridoma can be produced in the manner described in the Journal of Surgical Research, 38, 383-390 (1985); the hybridoma MM2-986 used here was obtained from Mr. Tsuyoshi Takami, one of the authors of that paper] was transplanted into the peritoneal cavity of pristane-treated mice (CBF1). MM2-986 was intraperitoneally proliferated; ascites fluid containing anti-B16-melanoma antibody was then collected. From this ascited fluid, anti-B16-melanoma antibody was obtained using a monoclonal antibody purification column ABx (J.T. Baker Inc., USA).

Example 3

A conjugate was obtained by reacting EMCS and IL-2 in a molar ratio of 4 to 1 with the total concentration limited so that the EMCS concentration was 0.05 μmol·mℓ in the manner described in Example 1.

### Example 4

A conjugate was obtained by reacting EMCS and IL-2 in the manner described in Example 1 in a molar ratio of 10 to 1 with the total concentration limited so that the EMCS concentration was 1.3 μmol·mℓ.

### Example 5

A conjugate was obtained by reacting EMCS and IL-2 in the manner described in Example 1 in a molar ratio of 100 to 1 with the total concentration limited so that the EMCS concentration was 6.5 μmol·mℓ.

### Example 6

A conjugate was obtained in the manner described in Example 5 but SPDP was used in place of EMCS.

### Example 7

A conjugate was obtained in the manner described in Example 4 but an anti-mouse-B16-melanoma monoclonal antibody produced by hybridoma MM2-9B6 was used in place of human IgG.

### Example 8

A conjugate is obtained in the manner described in Example 7 but Fab' of anti-mouse-B16-melanoma monoclonal antibody is used in place of anti-mouse-B16-melanoma monoclonal antibody.

Note that Fab' of anti-mouse-B16-melanoma monoclonal antibody is produced as follows:

An anti-B16-melanoma monoclonal antibody (mouse IgG) which is produced by hybridoma MM2-9B6 is reacted with pepsine at 37° C, pH 4.5 for 16 hours. After completion of the reaction, the reaction mixture is adjusted to pH 8.0 and passed through Sephadex G-150 (Pharmacia) to separate and purify F(ab')₂. After concentration, the F(ab')₂ is subjected to buffer exchange with a 0.1 M acetate buffer solution (pH 5.0) and is reduced with mercaptoethylamine, followed by Fab' separation and purification using Sephadex G-25 (produced by Pharmacia Co:).

### Example 9

A conjugate was obtained in the manner described in Example 1 but Fab' of human IgG was used in place of human IgG. Thus obtained conjugate showed IL-2 activity.

Note that human IgG Fab' was produced as follows:

Human IgG was reacted with pepsine at 37° C, pH4.5 for 16 hours. After completion of the reaction, the reaction mixture was adjusted to pH 6.0 and the mixture was passed through Sephadex S-200 (Pharmacia) to separate and purify F(ab')₂. After concentration, F(ab')₂ was subjected to buffer exchange with a 0.1 M phosphate buffer solution (pH 6.0) and was reduced with 0.1 M mercaptoethylamine. Thus obtained was subjected to separation by passing through Sephadex G-25 (Pharmacia) to give Fab'.

### Example 10

Ten mg of mouse IgG was reacted with 80 μg of N-succinimidyl-S-acetylthio-acetate (SATA) in a phosphate buffer (pH 6.0). The reaction mixture was subjected to purification using Sephadex G-25, and thus obtained purified product was subjected to reduction in an 1 M aqueous solution of hydroxylamine to emerge a thiol group in mouse IgG. After purifying with Sephadex G-25, the mouse IgG was reacted with 2 mg of IL-2 obtained by a genetic engineering technique (rIL-2), and EMCS to give a conjugate of mouse IgG and rIL-2.

The conjugate was purified employing Sephacryl S-200 column and then column chromatography using anti-IL-2 antibody. The activity of the conjugate was about 7 μg/ml, by measuring EIA sandwich method. When the aqueous solution of the conjugate (total amount: 900 ng) was intraveneously administered to mouse, it was retained in a blood, and blood plasma showed IL-2 activity as 10 ng/ml after 3 days.

### Example 11

F(ab')2 fragment of human IgG was obtained by treating human IgG with pepsine by the manner described in Journal of Immunoassay, 4, 209-327 (1983).

Four mg of human IgG F(ab')2 was reacted with 32 μg of N-succinimidyl-S-acetylthio-acetate (SATA) in a phosphate buffer (pH 6.0). The reaction mixture was subjected to purification using Sephadex G-25, and thus obtained purified product was subjected to reduction in an 1 M aqueous solution of hydroxylamine to emerge a thiol group in mouse IgG. After purifying with Sephadex G-25, the mouse IgG was reacted with 1 mg of IL-2 obtained by a genetic engineering technique (rIL-2) and EMCS to give a conjugate of human IgG F-(ab')2 and rIL-2.

The conjugate was purified by employing Sephacryl S-200 column and then affinity column chromatography using anti-IL-2 antibody. The activity of the conjugate was about 2 μg/ml, by measuring EIA sandwich method. When the aqueous solution of the conjugate (total amount: 300 ng) was intraveneously administered to mouse, it was retained in a blood, and the blood plasma showed IL-2 activity as 10 ng/ml after 3 days.

## Claims

1. A modified interleukin-2 (IL-2), in which IL-2 and immunoglobulin (Ig) or its fragment having an antibody activity are chemically bound with each other.

2. A modified IL-2 as claimed in Claim 1, wherein an amino group of IL-2 and a mercapto group resulting from a reduction of immunoglobulin or its fragment having an antibody activity are bound via a crosslinking agent.

3. A modified IL-2 as claimed in Claim 1, wherein an amino group of IL-2 and a thiol group which has been introduced to an amino group of immunoglobulin or its fragment having an antibody activity are bound via a crosslinking agent.

4. A modified IL-2 as claimed in Claim 1, wherein IL-2 is a recombinant human IL-2.

5. A modified IL-2 as claimed in Claim 1, wherein Ig is IgG.

6. A modified IL-2 as claimed in Claim 1, wherein Ig fragment having an antibody activity is employed.

7. A modified IL-2 as claimed in Claim 6, wherein Ig fragment is Fab, Fab' or F(ab')2.

8. A modified IL-2 as claimed in Claim 6, wherein Ig fragment is Fab'.

9. A modified IL-2 as claimed in Claim 6, wherein Ig fragment is F(ab')2.

10. A method for producing a modified interleukin-2 (IL-2), which comprises binding via a crosslinking agent an amino group of IL-2 and a mercapto group resulting from a reduction of immunoglobulin or its fragment having an antibody activity.

11. A method as claimed in Claim 10, wherein the crosslinking agent is N-(ε-maleimidocaproyloxy)succinimide (EMCS).

12. A method for producing a modified interleukin-2 (IL-2), which comprises binding via a crosslinking agent an amino group of IL-2 and a thiol group which has been introduced to the amino group of immunoglobulin or its fragment having an antibody activity.

13. A method as claimed in Claim 12, wherein the crosslinking agent is N-(ε-maleimidocaproyloxy)succinimide (EMCS).

14. A method as claimed in Claim 12, wherein the crosslinking agent is N-(ε-maleimidocaproyloxy)succinimide (EMCS) and the thiol group-introducing agent is N-succimidyl-s-acetylthioacetate (SATA).

15. A modified interleukin-2 (IL-2) produced by binding via a crosslinking agent an amino group of IL-2 and a mercapto group resulting from a reduction of immunoglobulin or its fragment having an antibody activity.

16. A modified interleukin-2 (IL-2) produced by binding via a crosslinking agent an amino group of IL-2 and a thiol group which has been introduced to an amino group of immunoglobulin or its fragment having an antibody activity.

Claims for the Following Contracting States: GR, ES

1. A method for producing a modified interleukin-2 (IL-2), which comprises binding via a crosslinking agent an amino group of IL-2 and a mercapto group resulting from a reduction of immunoglobulin or its fragment having an antibody activity.

2. A method as claimed in Claim 1, wherein IL-2 is a recombinant human IL-2.

3. A method as claimed in Claim 1, wherein Ig is IgG.

4. A method as claimed in Claim 1, wherein Ig fragment having an antibody activity is employed.

5. A method as claimed in Claim 4, wherein Ig fragment is Fab, Fab' or F(ab')2.

6. A method as claimed in Claim 4, wherein Ig fragment is Fab'.

7. A method as claimed in Claim 4, wherein Ig fragment is F(ab')2.

8. A method as claimed in Claim 1, wherein the crosslinking agent is N-(ε-maleimidocaproyloxy)succinimide (EMCS).

9. A method for producing a modified interleukin-2 (IL-2), which comprises binding via a crosslinking agent an amino group of IL-2 and a thiol group which has been introduced employing a thiol group-introducing agent to the amino group of immunoglobulin or its fragment having an antibody activity.

10. A method as claimed in Claim 9, wherein IL-2 is a recombinant human IL-2.

11. A method as claimed in Claim 9, wherein Ig is IgG.

12. A method as claimed in Claim 9, wherein Ig fragment having an antibody activity is employed.

13. A method as claimed in Claim 12, wherein Ig fragment is Fab, Fab' or F(ab')$_2$ of Ig.

14. A method as claimed in Claim 12, wherein Ig fragment is Fab'.

15. A method as claimed in Claim 12, wherein Ig fragment is F(ab')$_2$.

16. A method as claimed in claim 9, wherein the crosslinking agent is N-($\epsilon$-maleimidocaproyloxy)succinimide (EMCS) and the thiol group-introducing agent is N-succimidyl-s-acetyl-thioacetate (SATA).

Fig.1


1
Ala Pro Thr Ser Ser Ser Thr Lys Lys Thr Gln Leu Gln

                    20
Leu Glu His Leu Leu Leu Asp Leu Gln Met Ile Leu Asn


Gly Ile Asn Asn Tyr Lys Asn Pro Lys Leu Thr Arg Met

40
Leu Thr Phe Lys Phe Tyr Met Pro Lys Lys Ala Thr Glu

                    60
Leu Lys His Leu Gln Cys Leu Glu Glu Glu Leu Lys Pro


Leu Glu Glu Val Leu Asn Leu Ala Gln Ser Lys Asn Phe

    80
His Leu Arg Pro Arg Asp Leu Ile Ser Asn Ile Asn Val

                  100
Ile Val Leu Glu Leu Lys Gly Ser Glu Thr Thr Phe Met


Cys Glu Tyr Ala Asp Glu Thr Ala Thr Ile Val Glu Phe

    120
Leu Asn Arg Trp Ile Thr Phe Cys Gln Ser Ile Ile Ser

    133
Thr Leu Thr

Fig. 2

Fig. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,Y | EP-A-0 305 967 (CIBA GEIGY) <br> * Page 2, lines 15-25; page 15, line 1 - page 17, line 24 * <br> --- | 1-10 | A 61 K 37/02 <br> A 61 K 47/48 <br> A 61 K 39/395 |
| Y | EP-A-0 269 455 (TAKEDA CHEMICAL INDUSTRIES) <br> * Page 2, line 50 - page 3, line 27 * <br> --- | 1-10 | |
| Y | EP-A-0 256 714 (CETUS CORP.) <br> * Page 3, line 11 - line 42 * <br> --- | 1-10 | |
| D | WO-A-8 500 974 (HYBRITECH) <br> ----- | | |

TECHNICAL FIELDS SEARCHED (Int. Cl.5)

A 61 K

The present search report has been drawn up for all claims

| Place of search | Date of completion of the search | Examiner |
|---|---|---|
| THE HAGUE | 13-02-1990 | TURMO Y BLANCO C.E. |

EPO FORM 1503 03.82 (P0401)